# EUROPEAN PATENT APPLICATION

(11) **EP 4 501 375 A2**
(43) Date of publication of application: **05.02.2025**
(21) Application number: 24220508.6
(22) Date of filing: 18.09.2020
(51) Int. Cl.: A61M 5/142

(54) **FLUID DIVERTING DEVICE FOR AN APPARATUS FOR EXTRACORPOREAL TREATMENT OF BLOOD AND BLOOD SET PROVIDED WITH SAID FLUID DIVERTING DEVICE**

(30) Priority: 13.12.2019 EP 19216147
(62) Divisional of application: 20772066.5
(71) Applicant: Gambro Lundia AB, 226 43 Lund (SE)
(72) Inventor: VERNIN, Guillaume, 69330 MEYZIEU (FR)
(74) Representative: PGA S.p.A., Milano, Succursale di Lugano

(57) **Abstract**

A fluid diverting device for an apparatus for extracorporeal treatment of blood is configured to be placed in-line between a main portion (22) of the apparatus (1) and a vascular access of a patient (P) and comprises: a substantially H-shaped conduits assembly comprising a withdrawal conduit (23), a return conduit (24) and at least one bridging conduit (25, 125) connecting the withdrawal conduit (23) to the return conduit (24). The withdrawal conduit (23) is connectable upstream and downstream to a withdrawal line (6) of the apparatus (1), the return conduit (24) is connectable upstream and downstream to a return line (7) of the apparatus (1). A plurality of valves (26, 27, 28, 29, 30, 32) or distributors (201, 202) operate on the withdrawal conduit (23), on the return conduit (24) and on the at least one bridging conduit (25) and are configured to divert a flow of liquid and/or blood without disconnecting the patient (P).

## Description

### TECHNICAL FIELD

The invention relates to a fluid diverting device for an apparatus for extracorporeal treatment of blood and to a blood set for an apparatus for extracorporeal treatment of blood provided with this fluid diverting device. Extracorporeal blood treatment involves removing blood from a patient, treating the blood externally to the patient, and returning the treated blood to the patient. Extracorporeal blood treatment is typically used to extract undesirable matter or molecules from the patient's blood and add desirable matter or molecules to the blood. Extracorporeal blood treatment is used with patients unable to effectively remove matter from their blood, such as when a patient has suffered temporary or permanent kidney failure. These patients and other patients may undergo extracorporeal blood treatment to add or remove matter to their blood, to maintain an acid/base balance or to remove excess body fluids, or to perform extracorporeal gas exchange processes, for example.

For instance, in a haemodialysis treatment a patient's blood and a treatment liquid approximately isotonic with blood flow are circulated in a respective compartment of haemodialyser, so that, impurities and undesired substances present in the blood (urea, creatinine, etc.) may migrate by diffusive transfer from the blood into the treatment liquid. The ion concentration of the treatment liquid is chosen so as to correct the ion concentration of the patient's blood. In a treatment by haemodiafiltration, a convective transfer by ultrafiltration, resulting from a positive pressure difference created between the blood side and the treatment-liquid side of the membrane of a haemodiafilter, is added to the diffusive transfer obtained by dialysis. Hemoperfusion, oxygenation or decarboxylation treatments are also known.

A vascular access is used to remove the patient's blood so that it is filtered through the blood treatment unit (dialyzer, filter) and then returned to the patient. In case of chronic treatments, the vascular access may be arterio-venous. Two needles may be inserted into the vein, one to draw blood and one to return it. The orientation of the needles takes the normal flow of the blood into account. The "arterial" needle draws blood from the "upstream" location while the "venous" needle returns blood "downstream". In case of acute treatments, the vascular access may be veno-venous, i.e. using a double-lumen catheter inserted in a central vein. The blood treatment unit comprises pressure monitoring devices, as to check, for example, for circuit obstructions.

### BACKGROUND OF THE INVENTION

In the field of extracorporeal blood treatments and therapies, some problems reported by users are issues at the vascular access.

Access Extremely Negative (AEN) is one of the most encountered alarm in the field, mainly linked to catheter tip stuck against blood vessel's wall or, more rarely, to clots obstructing the access way of the catheter. When this alarm relates to catheter positioning inside the vein, the nurse has to get the access pressure back closer to an atmospheric level, e.g. through the use of a Y-connector connected to the access line, unstick the catheter tip from the vein wall and then try to move the catheter inside the vein and resume the treatment.

Another drawback is that at the end of the treatment, when blood is to be returned to the patient, one operator has to disconnect the patient access and connect a saline bag to the set's access line, in aseptic conditions, and another operator has to operate on the monitor of the machine in non-aseptic conditions. Therefore, two operators are needed for one patient.

Another drawback is that, as per the blood return described above, both saline and blood recirculation procedures currently require sterile and non-sterile interventions and the manual connection of supplementary devices (Y-connector, saline bag).

In all the cases detailed above, the operator has to handle the vascular access, to disconnect and eventually reconnect the same to the patient. All this implies operator workload and increased probability of occurrence for non-aseptic handling.

Furthermore, the use of a Y-connector connected to the access line involves further risks. On the access side, the user may forget to clamp the Y saline line prior to resuming the treatment. In case of blood pump stop and slightly negative patient access pressure, the saline bag content would be sucked into the catheter, causing air ingress leading to set clotting (or potentially air embolism).

Additional modules, configured to be placed in-line between the blood treatment apparatus and the patient vascular access and configured to divert the flow of blood without necessarily disconnecting the patient, are also known.

For instance, document US 3,626,938 discloses a shunt valve device which is designed to be mounted permanently on a body member of a person and permanently connected to the artery and vein of the person. The device is provided with valve means for selectively connecting the artery and vein to an artificial kidney or the like. The device further has rotary shunt means for directing the flow of blood back to the body member when the device is not operatively connected to the artificial kidney.

Document US 5,894,011 discloses a device for selectively controlling the direction of blood flow to and from the patient during hemodialysis. The device comprises two interlocking disks that rotate in relation to each other without separating. The two disks have fluid fittings that allow the blood lines attached to the patient to connect to one of the disks and the blood inlet and outlet for the hemodialysis machine to connect to the other. The center of each fluid fitting is a channel that aligns to a corresponding channel in the other disk. The disks rotate between two fixed relative positions, referred to herein as preferred alignments. The preferred alignments are such that the line drawing blood from the patient in the first preferred alignment becomes the line returning blood to the patient in the second preferred alignment, and the line returning blood to the patient in the first preferred alignment becomes the line drawing blood from the patient in the second preferred alignment. A bypass channel allows blood to flow from the outlet to the inlet of the hemodialysis machine when the device is in neither of its two preferred alignments.

These devices allow a limited number of flow path configurations and, due to their structure and geometry of the ducts, may cause pressure drops and/or clotting.

It is therefore an object of the present invention to provide a fluid diverting device for an apparatus for extracorporeal treatment of blood, wherein the fluid diverting device is configured to divert the flow of blood or other fluid/s in order to perform a plurality of procedures (e.g. all the steps of the treatment, pre and post treatment and possible troubleshooting procedures) without necessarily disconnecting the patient.

It is an object of the present invention to provide a fluid diverting device which may be easily interposed between the apparatus for extracorporeal treatment of blood and the patient, featuring various functional components and able to be set in a plurality of configurations to perform the plurality of procedures.

In particular, it is an object providing a fluid diverting device able to provide easy and safe AEN troubleshooting, blood return at the treatment end, blood recirculation, saline recirculation.

It is a further object providing a fluid diverting device allowing to control its configurations in automated manner.

It is also an object of the present invention to provide a fluid diverting device enabling offering the device features as a supplemental option with respect to the apparatus.

It is a further object providing a fluid diverting device allowing a single person to handle the patient's vascular access in safe manner and in aseptic conditions.

It is a further object providing a fluid diverting device offering the possibility to have some commands located conveniently nearby the patient access.

It is a further object providing a fluid diverting device offering the possibility to operate disinfection procedures in aseptic conditions.

It is a further object providing a fluid diverting device which is structurally simple, cost effective and reliable.

It is a further object providing a fluid diverting device able to prevent clotting and/or to avoid high pressure losses and/or requiring minimal amounts of fluid needed for flushing said fluid diverting device.

### SUMMARY

At least one of the above objects is substantially reached by a fluid diverting device for an apparatus for extracorporeal treatment of blood and by an apparatus for extracorporeal treatment of blood according to one or more of the appended claims.

A fluid diverting device for an apparatus for extracorporeal treatment of blood, an apparatus for extracorporeal treatment of blood and a method for diverting a flow of liquid and/or blood in an apparatus for extracorporeal treatment of blood according to aspects of the invention and capable of achieving one or more of the above objects are here below described.

A 1^{st} aspect concerns a fluid diverting device for an apparatus for extracorporeal treatment of blood. The fluid diverting device is configured to be placed in-line between a blood set of an apparatus for extracorporeal treatment of blood and a vascular access of a patient. The fluid diverting device comprises: a substantially H-shaped conduits assembly comprising a withdrawal conduit, a return conduit and at least a bridging conduit connecting or configured to connect the withdrawal conduit to the return conduit; wherein the withdrawal conduit is connected or connectable upstream and downstream to a withdrawal line of the blood set, wherein the return conduit is connected or connectable upstream and downstream to a return line of the blood set; a plurality of valves or distributors operating on the withdrawal conduit and/or on the return conduit and/or on the at least a bridging conduit and configured to divert a flow of liquid and/or blood without disconnecting the patient.

A 2^{nd} aspect concerns a blood set for an apparatus for extracorporeal treatment of blood comprising: a blood treatment unit; an extracorporeal blood circuit coupled to the blood treatment unit and comprising a blood withdrawal line and a blood return line connectable to a vascular access of a patient; a fluid diverting device according to the preceding 1^{st} aspect or to one or more of the following aspects, wherein the fluid diverting device is placed or is configured to be placed in-line between a main portion of the blood set of apparatus for extracorporeal treatment of blood and the vascular access of the patient. The apparatus comprises a blood pump configured to be coupled to a pump section of the extracorporeal blood circuit.

In a further independent aspect, an apparatus for extracorporeal treatment of blood is provided comprising the blood set of the previous aspect.

A 3^{rd} aspect concerns a method for diverting a flow of liquid and/or blood in an apparatus for extracorporeal treatment of blood without disconnecting the patient, comprising: placing in-line a fluid diverting device between the apparatus for extracorporeal treatment of blood and a vascular access of a patient, wherein the fluid diverting device is according to the preceding 1^{st} aspect or to one or more of the following aspects.

In a 4^{th} aspect according to any one of the preceding aspects, the withdrawal conduit is substantially parallel to the return conduit.

In a 5^{th} aspect according to any one of the preceding aspects, the bridging conduit is at least in part transversal, optionally, perpendicular to the withdrawal conduit and to the return conduit.

In a 6^{th} aspect according to the preceding aspects, the withdrawal conduit and/or the return conduit and/or the bridging conduit is/are tubes, optionally of plastic, optionally at least in part straight.

In a 7^{th} aspect according to any one of the preceding aspects, the plurality of valves comprises at least a withdrawal valve operating on the withdrawal conduit, at least a return valve operating on the return conduit and at least a bridging valve operating on the bridging conduit.

In an 8^{th} aspect according to any one of the preceding aspects, the valves of the plurality of valves are clamps or pinches, optionally at least one of the plurality of valves is a rotating clamp or pinch.

In an 8^{th} bis aspect according to the previous aspect, the rotating clamp or pinch comprises a rotating body and an abutment element.

In an 8^{th} ter aspect according to the previous aspect, the rotating body is rotatable between a clamping position (valve closed), in which the rotating element is close to the abutment element to close a portion of a tube between said rotating element and said abutment element, and a release position, in which the rotating element is spaced from the abutment element (valve open), to open said portion of tube.

In an 8^{th} quater aspect according to the previous aspect, at least two valves of the plurality of valves comprise one common rotating body and respective abutment elements. One rotating body and two abutment elements define two valves.

In an 8^{th} quinquies aspect according to the previous aspect, the rotating body is rotatable between:
a first clamping position (one of the two valves closed and the other open), in which the rotating element is close to one of the two abutment elements to close a portion of a tube between said rotating element and said abutment element;
a second clamping position (one of the two valves closed and the other open), in which the rotating element is close to the other of the two abutment elements to close a portion of another tube between said rotating element and said abutment element; a release position, in which the rotating element is spaced from the abutment elements (both valves open), to open both portions of tubes.

In a 9^{th} aspect according to the preceding aspect 7 or 8, said at least a withdrawal valve comprises a first withdrawal valve and a second withdrawal valve operating on the withdrawal conduit.

In a 10^{th} aspect according to the preceding aspect 9, the first withdrawal valve and the second withdrawal valve are placed on opposite sides with respect to a junction of the bridging conduit to the withdrawal conduit.

In a 11^{th} aspect according to the preceding aspect 9 or 10, the first withdrawal valve is placed upstream, with respect to a flow of blood during treatment, a junction of the bridging conduit to said withdrawal conduit.

In a 12^{th} aspect according to the preceding aspect 9, 10 or 11, the second withdrawal valve is placed downstream, with respect to a flow of blood during treatment, a junction of the bridging conduit to said withdrawal conduit.

In a 13^{th} aspect according to aspect 7 or to any one of the preceding aspects 8 to 12 when according to aspect 7, the return valve is placed downstream a junction of the bridging conduit to the return conduit with respect to a flow of blood during treatment.

In a 14^{th} aspect according to aspect 7 or to any one of the preceding aspects 8 to 12 when according to aspect 7, the return valve comprises a first return valve and a second return valve operating on the return conduit.

In a 15^{th} aspect according to the preceding aspect 14, the first return valve and the second return valve are placed on opposite sides with respect to a junction of the bridging conduit to the return conduit.

In a 16^{th} aspect according to the preceding aspect 14 or 15, the first return valve is placed downstream, with respect to a flow of blood during treatment, a junction of the bridging conduit to said return conduit.

In a 17^{th} aspect according to the preceding aspect 14, 15 or 16, the second return valve is placed upstream, with respect to a flow of blood during treatment, a junction of the bridging conduit to said return conduit.

In a 18^{th} aspect according to aspect 7 or to any one of the preceding aspects 8 to 17 when according to aspect 7, said at least a bridging valve comprises a first bridging valve and a second bridging valve operating on the bridging conduit.

In a 19^{th} aspect according to the preceding aspect 18, the first bridging valve is placed closer to the return conduit (closer than the second bridging valve) and the second bridging valve is placed closer to the withdrawal conduit (closer than the first bridging valve).

In a 20^{th} aspect according to the preceding aspect 18 or 19, the bridging conduit has an access point located between the first bridging valve and the second bridging valve and suitable to connect a liquid source; optionally the access point comprises an access valve.

In a 20^{th} bis aspect according to any of aspects 18 to 20 and to any of aspects 8 to 8 quinquies, the first bridging valve and the return valve comprise the common rotating body and the respective abutment elements.

In a 20^{th} ter aspect, according to any of aspects 18 to 20 and to any of aspects 8 to 11, the second bridging valve and the first withdrawal valve comprise the common rotating body and the respective abutment elements.

In a 21^{st} aspect according to the preceding aspect 20, the liquid source is a saline source.

In a 22^{nd} aspect according to the preceding aspect 20 or 21, the liquid source is a syringe, e.g. of 50 ml, or a bag, e.g. of 500 ml.

In a 23^{rd} aspect according to the preceding aspect 20 or 21 or 22, the fluid diverting device comprises a liquid source, optionally a syringe or a bag, connected or connectable to the access point.

In a 24^{th} aspect according to any of the preceding aspects 1 to 23, the fluid diverting device comprises a holder configured to hold the substantially H-shaped conduits assembly and the plurality of valves, optionally the holder has seats to accommodate the substantially H-shaped conduits assembly and the plurality of valves.

In a 25^{th} aspect according to the preceding aspects 24 when according to aspect 22 or 23, the holder is configured to hold the liquid source, optionally the syringe or the bag, optionally the holder has seats to accommodate the liquid source, optionally the syringe or the bag; optionally, the holder comprising a device for pressurizing the liquid source, optionally the syringe, like a spring or an actuator, or the bag.

In a 26^{th} aspect according to any of the preceding aspects 1 to 25, the fluid diverting device is at least in part disposable.

In a 26^{th} bis aspect according to any of the preceding aspects 1 to 25, the fluid diverting device is an add-on module.

In a 26^{th} ter aspect according to any of the preceding aspects 1 to 26, the fluid diverting device is placed on a main portion of the apparatus for extracorporeal treatment of blood.

In a 26^{th} quater aspect according to the preceding aspect, the fluid diverting device is placed on a side of the main portion.

In a 26^{th} quinquies aspect according to the preceding aspect, the main portion comprises at least a casing for a control unit, the control unit, a user interface, the blood pump, the effluent pump, the dialysis fluid pump, the infusion pumps and devices to mount a disposable set (comprising the blood treatment unit, the extracorporeal blood circuit, the infusion circuit and the fluid circuit) to the main portion.

In a 27^{th} aspect according to the preceding aspect 7 or to any of aspects 8 to 26 quinquies when according to aspect 7, the plurality of valves is configured to be arranged at least in a treatment configuration, in which said at least a withdrawal valve and said at least a return valve are open and said at least a bridging valve is closed, to perform treatment of the patient.

In a 28^{th} aspect according to the preceding aspect 9 or to any one of the preceding aspects 10 to 27 when according to aspect 9, the plurality of valves is configured to be arranged at least in a recirculation configuration, in which the return valve is closed, the first withdrawal valve is closed, the second withdrawal valve is open and said at least a bridging valve is open, so that blood or liquid recirculates in the blood treatment unit of the apparatus without disconnecting the patient.

In a 29^{th} aspect according to the preceding aspect 18 or to any one of the preceding aspects 19 to 28 when according to aspect 18, the plurality of valves is configured to be arranged in a return branch flushing configuration, in which the first bridging valve is open, the second bridging valve is closed and the liquid source is connected to the access point to inject liquid and flush a branch of the return conduit placed downstream a junction of the bridging conduit to the return conduit and optionally to flush a catheter at the vascular access of a patient.

In a 30^{th} aspect according the preceding aspect 29, in the return branch flushing configuration, a monitor valve on the blood return line (located between the apparatus and the fluid diverting device) is closed or, when aspect 18 is according to aspect 14, the second return valve is closed; wherein the first withdrawal valve, the second withdrawal valve, the return valve (or the first return valve) and the access valve are open.

In a 31^{st} aspect according the preceding aspect 18 or to any one of the preceding aspects 19 to 30 when according to aspect 18, the plurality of valves is configured to be arranged in a withdrawal branch flushing configuration, in which the first bridging valve is closed, the second bridging valve is open and the liquid source is connected to the access point to inject liquid and flush a branch of the withdrawal conduit placed upstream a junction of the bridging conduit to the withdrawal conduit and optionally to flush a catheter at the vascular access of a patient.

In a 32^{nd} aspect according the preceding aspect 31, in the withdrawal branch flushing configuration, the second withdrawal valve is closed.

In a 33^{rd} aspect according the any of the preceding aspects 1 to 32, the valves of the plurality of valves or the distributors are operatively connected or connectable to a control unit, optionally of the apparatus for extracorporeal treatment of blood, and/or the fluid diverting device comprises a control unit operatively connected to the plurality of valves or distributors; the control unit being configured for commanding, optionally automatically, the configurations of the plurality of valves or distributors.

In a 34^{th} aspect according to the preceding aspect 33, the fluid diverting device is connected or configured to be connected to the control unit of the apparatus for extracorporeal treatment of blood, optionally through wired or wireless communication.

In a 35^{th} aspect according to any of the preceding aspects 1 to 34, the fluid diverting device comprises a power supply or is connected to a power supply of the apparatus for extracorporeal treatment of blood.

In a 36^{th} aspect according to any of the preceding aspects 1 to 35, the fluid diverting device comprises an attachment device configured to install said fluid diverting device, e.g. to clothing of a patient or to a bed for the patient or to a main portion of the apparatus for extracorporeal treatment of blood, optionally in a removable manner.

In a 37^{th} aspect according to any of the preceding aspects 1 to 36, the fluid diverting device comprises quick connectors, optionally Luer connectors, placed at extremities of the withdrawal conduit and of the return conduit. In a 38^{th} aspect according to any of the preceding aspects 1 to 36, the fluid diverting device comprises a further access point on the return conduit, e.g. for calcium infusion.

In a 39^{th} aspect according to any of the preceding aspects 1 to 37, the fluid diverting device comprises at least one sensor active on the H-shaped conduits assembly, optionally on the return conduit and/or on the withdrawal conduit and/or on the bridging conduit; said at least one sensor being connected or configured to be connected to a control unit of the apparatus for extracorporeal treatment of blood or of the fluid diverting device; said at least one sensor may be a pressure sensor, a flowmeter, an air detector.

In a 40^{th} aspect according to one or more of the previous aspects 7 to 23, a control unit of the apparatus for extracorporeal treatment of blood or of the fluid diverting device is configured for commanding execution of a task for diverting a flow of liquid and/or blood, said task or the method for diverting a flow of liquid and/or blood comprises the following steps:
- keeping said at least a withdrawal valve, optionally the first withdrawal valve and the second withdrawal valve, open and said at least a return valve open and keeping said at least a bridging valve, optionally the first bridging valve and the second bridging valve, closed to perform treatment of the patient;
- optionally, opening regularly the first bridging valve and the second bridging valve and the access valve, to flush with fluid and prevent coagulation; or opening regularly the first bridging valve and the second bridging valve while the access valve is closed, to create a short recirculation and prevent coagulation.

In a 41^{st} aspect according to the previous aspect, in case of Access Extremely Negative (AEN) alarm, said task or the method for diverting a flow of liquid and/or blood comprises the following steps:
- stopping the blood pump;
- optionally, opening the second bridging valve and the access valve enabling liquid to be sucked from liquid source until access pressure reaches an approximately atmospheric level or opening the first bridging valve and the second bridging valve to create connection between withdrawal conduit and return conduit and normalize pressure; then
- keeping the first bridging valve closed, closing the second withdrawal valve and keeping the access valve, the second bridging valve and the first withdrawal valve open to enable liquid from liquid source to flush the vascular access of the patient backwards, to dislodge catheter tip from vessel's wall.

In a 42^{nd} aspect according to the previous aspect 40, said task or the method for diverting a flow of liquid and/or blood comprises the following steps:
- optionally, opening the first bridging valve and the access valve and closing the first withdrawal valve and the second withdrawal valve and a monitor valve on the blood return line (located between the apparatus and the fluid diverting device) while the return valve is open and the second bridging valve is closed, to flush with liquid at least part of the return conduit;
- optionally, closing the first bridging valve and the second withdrawal valve and opening the second bridging valve while the return valve, the access valve and the first withdrawal valve are open, to flush with liquid at least part of the withdrawal conduit;
- closing the return valve, the first withdrawal valve and the access valve and opening the second withdrawal valve while the first bridging valve and the second bridging valve are open to recirculate blood in the blood treatment unit of the apparatus.

In a 43^{rd} aspect according to the previous aspect 40, said task or the method for diverting a flow of liquid and/or blood comprises the following steps:
- closing the first withdrawal valve and opening the second bridging valve and the access valve while the return valve and the second withdrawal valve are open;
- pumping liquid until all blood is returned to the patient;
- closing the return valve and opening the first bridging valve while the second bridging valve is open, the second withdrawal valve is open and the first withdrawal valve is closed, to recirculate liquid in the blood treatment unit of the apparatus.

In a 44^{th} aspect according to any of the previous aspects 1 to 43, the blood treatment unit comprises a primary chamber and a secondary chamber separated by a semi-permeable membrane, wherein the blood withdrawal line is connected to an inlet of the primary chamber and the blood return line is connected to an outlet of the primary chamber. In particular, the blood treatment unit is part of a blood set of the apparatus.

In a 45^{th} aspect according to the previous aspect 44, an effluent fluid line is connected to an outlet of the secondary chamber and a dialysis fluid line is connected to an inlet of the secondary chamber and to a dialysis liquid source.

In a 45^{th} bis aspect, an infusion circuit comprising one or more infusion lines of a replacement fluid is connected to the extracorporeal blood circuit. In particular, the effluent fluid line and the infusion circuit are part of a blood set of the apparatus.

In a 45^{th} ter aspect according to aspect 45 bis and 20, at least one infusion line is connected to the access point and, optionally, an infusion fluid container is connected to the infusion line. The liquid source may be the infusion fluid container.

In a 45^{th} quater aspect according to aspect 45 ter, an infusion pump operates on the infusion line.

In a 45^{th} quinquies aspect according to aspect 45 ter, the at least one infusion line comprises an infusion line connected to the blood withdrawal line between the blood pump and the treatment unit.

In a 45^{th} sexies aspect according to aspect 45 ter, the at least one infusion line comprises a pre-blood pump infusion line connected to the access point of the fluid diverting device and/or to the blood withdrawal line between the vascular access and the fluid diverting device.

In a 45^{th} septies aspect according to the aspect 45 quinquies and/or 45 sexies, an infusion pump operates on the infusion line and/or an infusion pump operates on the pre-blood pump infusion line.

In a 45^{th} octies aspect according to aspect 45 sexies and/or 45 septies, at least an infusion valve operates on the pre-blood pump infusion line; optionally a first infusion valve or access valve and a second infusion valve; optionally the access valve is placed upstream a branch of the pre-blood pump infusion line connected to the access point of the fluid diverting device; optionally the second infusion valve is placed downstream a branch of the pre-blood pump infusion line connected to the access point of the fluid diverting device.

In a 45^{th} novies aspect according to the previous aspect, to aspects 9 to 12 and 8 to 8 quinquies, the second withdrawal valve and the first infusion valve or access valve comprise the common rotating body and the respective abutment elements.

In a 45^{th} decies aspect according to aspects 45 novies and 8 to 8 quinquies, the second infusion valve is a rotating clamp or pinch, optionally comprising the common rotating body and the abutment element.

In a 45^{th} undecies aspect according to any of aspects 9 to 12, an auxiliary withdrawal valve operates on the withdrawal conduit, wherein the auxiliary withdrawal valve is placed downstream, with respect to a flow of blood during treatment, the first withdrawal valve.

In a 45^{th} duodecies aspect according to aspects 45 octies and 45 undecies, the auxiliary withdrawal valve and the second infusion valve comprise the common rotating body and the respective abutment elements.

In a 46^{th} aspect according to any of the previous aspects 1 to 45, each of the blood withdrawal line and the blood return line has a respective end connector configured to be connected to the vascular access of the patient.

In a 47^{th} aspect according to any of the previous aspects 1 to 46, each of the withdrawal conduit and the return conduit has a respective end counter-connector configured to be coupled or coupled with a respective end connector of the blood withdrawal line and of the blood return line.

In a 48^{th} aspect according to any of the previous aspects 1 to 47, each of the withdrawal conduit and the return conduit has a respective end connector configured to be coupled or coupled with a respective end counter-connector of the vascular access of the patient.

In a 49^{th} aspect according to any of the previous aspects 1 to 48, an auxiliary bridging conduit connects or is configured to connect the withdrawal conduit to the return conduit.

In a 50^{th} aspect according to previous aspect 49, said auxiliary bridging conduit is arranged in parallel with respect to the bridging conduit.

In a 50^{th} bis aspect according to previous aspects 49 or 50, with respect to a flow of blood during treatment, the auxiliary bridging conduit is connected to the withdrawal conduit upstream the bridging conduit and is connected to the return conduit downstream the bridging conduit.

In a 51^{st} aspect according to previous aspects 49 or 50, an auxiliary pump operates on the auxiliary bridging conduit, wherein the auxiliary pump is at least configured to recirculate blood on a patient side.

In a 52^{nd} aspect according to previous aspect 49, 50 or 51, an auxiliary withdrawal valve operates on the withdrawal conduit and an auxiliary return valve operates on the return conduit.

In a 52^{nd} bis aspect according to previous aspect 52, with respect to a flow of blood during treatment, the auxiliary withdrawal valve is placed upstream a connection site of the auxiliary bridging conduit to the withdrawal conduit and the auxiliary return valve is placed downstream a connection site of the auxiliary bridging conduit to the return conduit.

In a 53^{rd} aspect according to previous aspect 52, the auxiliary withdrawal valve is placed upstream, with respect to a flow of blood during treatment, the first withdrawal valve; the auxiliary return valve is placed downstream, with respect to a flow of blood during treatment, the return valve.

In a 54^{th} aspect according to any of previous aspects 49 to 53, a first auxiliary bridging valve and a second auxiliary bridging valve operate on the auxiliary bridging conduit.

In a 55^{th} aspect according to aspect 54 when according to aspect 51, the first auxiliary bridging valve and the second auxiliary bridging valve are placed on opposite sides with respect to the auxiliary pump.

In a 56^{th} aspect according to aspects 52 and 54 and 8 bis to 8 quinquies, the auxiliary withdrawal valve and the second auxiliary bridging valve comprise the common rotating body and the respective abutment elements.

In a 57^{th} aspect according to aspects 52 and 54 and 8bis to 8 quinquies, the auxiliary return valve and the first auxiliary bridging valve comprise the common rotating body and the respective abutment elements.

In a 58^{th} aspect according to aspects 52 and 54 when according to aspect 29, 30 or 31, 32, in the withdrawal branch flushing configuration and/or in the return branch flushing configuration, the auxiliary withdrawal valve and the auxiliary return valve are open and the first auxiliary bridging valve and a second auxiliary bridging valve are closed.

In a 59^{th} aspect according to aspects 52 and 54 when according to aspect 28 or 29, in the recirculation configuration, the auxiliary withdrawal valve and the auxiliary return valve are open and the first auxiliary bridging valve and the second auxiliary bridging valve are open.

In a 60^{th} aspect according to the previous aspect 59 when aspects 52 and 54 are according to aspect 51, in the recirculation configuration, the auxiliary pump works to recirculate blood on the patient side.

In a 61^{st} aspect according to any of the previous aspect 49, 50 or 51 when according to any of aspects 1 to 6, a first distributor is operatively coupled to the withdrawal conduit, to the bridging conduit and to the auxiliary bridging conduit.

In a 62^{nd} aspect according to the previous aspect 61, a second distributor is operatively coupled to the return conduit, to the bridging conduit and to the auxiliary bridging conduit.

In a 63^{rd} aspect according to aspects 61 and 62, the first distributor and the second distributor are movable between: a first configuration corresponding to the treatment configuration and a second configuration corresponding to the recirculation configuration.

In a 64^{th} aspect according to aspect 63, in the first configuration the withdrawal conduit delimits a continuous passage connected to the withdrawal line, the return conduit delimits a continuous passage connected to the return line and the bridging conduit together with the auxiliary bridging conduit delimit a closed loop disconnected from the withdrawal line and from the return line.

In a 65^{th} aspect according to aspect 63 or 64, in the second configuration, the bridging conduit delimits a closed loop with the blood treatment unit to recirculate blood in the blood treatment unit and in which the auxiliary bridging conduit delimits a closed loop connected to the access device, to recirculate blood on a patient side.

In a 66^{th} aspect according to previous aspect 65, each of the first distributor and the second distributor comprises a rotating element provided with through passages configured to communicate with branches of the withdrawal conduit or of the return conduit and with the bridging conduit and the auxiliary bridging conduit.

In a 67^{th} aspect according to the previous aspect 66, the through passages comprise a first through passage and a second through passage.

In a 68^{th} aspect according to the previous aspect 67, in the first configuration, the first through passage of the first distributor connects a first branch and a second branch of the withdrawal conduit and the second through passage of the first distributor connects the bridging conduit to the auxiliary bridging conduit.

In a 69^{th} aspect according to any of the previous aspects 67 and 68, in the first configuration, the first through passage of the second distributor connects a first branch and a second branch of the return conduit and the second through passage of the second distributor connects the bridging conduit to the auxiliary bridging conduit. In a 70^{th} aspect according to any of the previous aspects 67 to 69, in the second configuration, the first through passage of the first distributor connects a first branch of the withdrawal conduit to the auxiliary bridging conduit and the second through passage of the first distributor connects a second branch of the withdrawal conduit to the bridging conduit.

In a 71^{st} aspect according to any of the previous aspects 67 to 70, in the second configuration, the first through passage of the second distributor connects a first branch of the return conduit to the bridging conduit and the second through passage of the second distributor connects a second branch of the return conduit to the auxiliary bridging conduit.

### DESCRIPTION OF THE DRAWINGS

Aspects of the invention are shown in the attached drawings, which are provided by way of non-limiting example, wherein:
Figure 1 shows a schematic diagram of an apparatus for extracorporeal treatment of blood comprising a fluid diverting device according to the invention;
Figure 2 shows an enlarged view of the fluid diverting device of Figure 1;
Figure 3 shows another embodiment of the fluid diverting device of the invention;
Figure 4 shows a variant of the fluid diverting device of Figure 2;
Figures 5 to 11 show respective working configurations of the fluid diverting device of Figure 1;
Figures 12 to 15 show flowcharts of respective operating modes of the of the fluid diverting device of the invention;
Figure 16 shows an apparatus for extracorporeal treatment of blood comprising a different embodiment of a fluid diverting device according to the invention;
Figures 17 to 19 show respective working configurations of the fluid diverting device of Figure 16;
Figures 20A and 20B show enlarged views of an element of the fluid diverting device of Figure 16 in respective working positions;
Figure 21 shows a variant of the apparatus for extracorporeal treatment of Figure 16;
Figures 22 to 25 show respective working configurations of the fluid diverting device of Figure 21;
Figure 26 is a flowchart of a respective operating mode of the of the apparatus of Figures 21 to 25;
Figure 27 shows an apparatus for extracorporeal treatment of blood comprising a different embodiment of a fluid diverting device according to the invention;
Figures 28A and 28B show enlarged views of an element of the fluid diverting device of Figure 27 in respective working positions;
Figures 29 and 30 show respective working configurations of the fluid diverting device of Figure 27;
Figure 30 is a flowchart of a respective operating mode of the apparatus of Figures 27 to 30;
Figure 31 is a flowchart of another operating mode of the apparatus of Figures 27 to 30.

### DETAILED DESCRIPTION

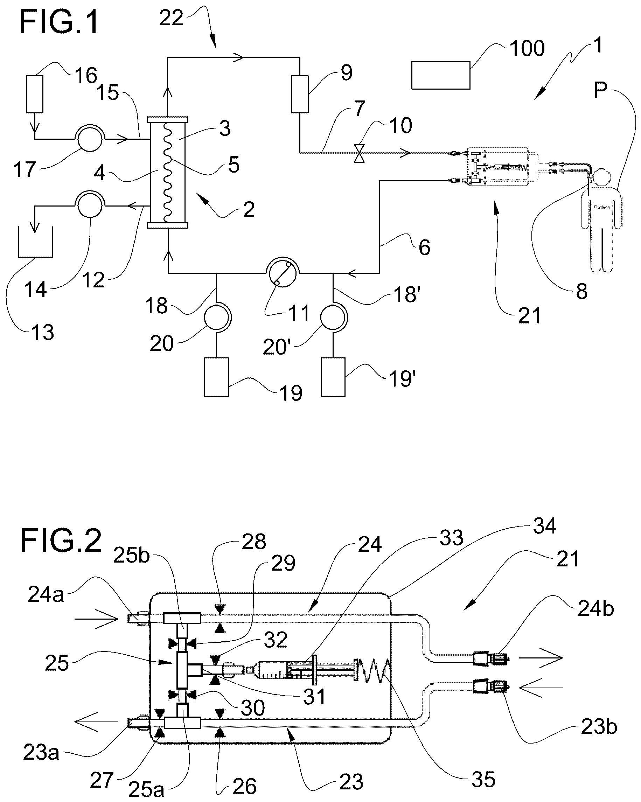

Non-limiting embodiments of a fluid diverting device - which may implement innovative aspects of the invention - for an apparatus 1 for extracorporeal treatment of blood are shown in Figures 2, 3, 4, 16, 21 and 26. In below description and in Figures 2, 3, 4, 16, 21 and 26 same components are identified by same reference numerals. An apparatus for the extracorporeal treatment of blood 1 is represented in Figure 1. The apparatus 1 of Figure 1 comprises a blood treatment unit 2 (such as a hemofilter, an ultrafilter, an hemodiafilter, a dialyzer, a plasmafilter and the like) having a primary chamber 3 and a secondary chamber 4 separated by a semi-permeable membrane 5. Depending upon the treatment, the semi-permeable membrane 5 of the blood treatment unit 2 may be selected to have different properties and performances.

A blood withdrawal line 6 is connected to an inlet of the primary chamber 3 and a blood return line 7 is connected to an outlet of the primary chamber 3. In use, the blood withdrawal line 6 and the blood return line 7 are connected to a needle or to a catheter or other access device 8 which is then placed in fluid communication with the patient "P" vascular system, such that blood may be withdrawn through the blood withdrawal line 6, flown through the primary chamber 3 and then returned to the patient's vascular system through the blood return line 7. An air separator, such as a deaeration chamber 9, may be present on the blood return line 7. Moreover, a monitor valve 10 may be present on the blood return line 7, downstream the deaeration chamber 9.

The blood flow through the blood lines is controlled by a blood pump 11, for instance a peristaltic blood pump, acting either on the blood withdrawal line 6 or on the blood return line 7. The embodiment of Figure 1 shows the blood pump 11 coupled to a pump section of the withdrawal line 6. A control unit 100 is connected and controls the blood pump 11 to regulate a blood flow rate.

An effluent fluid line 12 or spent dialysate line may be connected, at one end, to a fluid outlet of the secondary chamber 4 and, at its other end, to a waste which may be a discharge conduit or an effluent fluid container 13 collecting the effluent fluid extracted from the secondary chamber 4. An effluent pump 14 that operates on the effluent fluid line 13 under the control of the control unit 100 to regulate a flow rate of the effluent fluid through the effluent fluid line.

The apparatus of Figure 1 includes a dialysis fluid line 15 connected at one end with a fresh dialysis liquid source 16 and at its other end with a fluid inlet of the secondary chamber 4 of the treatment unit 2 for supplying fresh dialysis liquid to the secondary chamber 4. A dialysis fluid pump 17 is operative on the dialysis fluid line 15 under the control of the control unit 100 to supply fluid from the dialysis liquid source 16 to the secondary chamber 4 and to regulate the flow rate of the dialysis liquid.

The embodiment of Figure 1 further presents an infusion line 18 connected to the blood withdrawal line 6 between the blood pump 11 and the treatment unit 2. This infusion line 18 supplies replacement fluid from an infusion fluid container 19 connected at one end of the infusion line 18. Note that, alternatively or in addition to the infusion line 18, the apparatus of Figure 1 may include a post-dilution fluid line (not shown) connecting an infusion fluid container to the blood return line 7 and/or a pre-blood pump infusion line 18' with its own pre-blood pump infusion fluid container 19'. Furthermore, an infusion pump 20 operates on the infusion line 18 and an infusion pump 20' operates on the pre-blood pump infusion line 18' to regulate respective flow rates through the infusion lines 18, 18'. Also the infusion pumps 20, 20' are operative under the control of the control unit 100.

The effluent fluid line 12, the dialysis fluid line 15 and the secondary chamber 4 of the blood treatment unit 2 are part of a fluid circuit of the apparatus 1. The blood withdrawal line 6, the blood return line 7, the primary chamber 3 of the treatment unit 2 form part of an extracorporeal blood circuit of the apparatus 1. The infusion lines 18, 18' form part of an infusion circuit of the apparatus 1.

The control unit 100 may comprise a digital processor (CPU) with memory (or memories), an analogical type circuit, or a combination of one or more digital processing units with one or more analogical processing circuits. In the present description and in the claims it is indicated that the control unit 100 is "configured" or "programmed" to execute steps: this may be achieved in practice by any means which allow configuring or programming the control unit 100. For instance, in case of a control unit 100 comprising one or more CPUs, one or more programs are stored in an appropriate memory: the program or programs containing instructions which, when executed by the control unit 100, cause the control unit 100 to execute the steps described and/or claimed in connection with the control unit 100. Alternatively, if the control unit 100 is of an analogical type, then the circuitry of the control unit 100 is designed to include circuitry configured, in use, to process electric signals such as to execute the control unit 100 steps herein disclosed.

The control unit 100 may also be operatively connected to sensors (like flow sensors and/or pressure sensors) on the blood circuit and/or fluid circuit and/or infusion circuit. The control unit 100 is also operatively connected to clamps and valves, like the monitor valve 10. The control unit 100 may also be connected to a user interface, not shown, for instance a graphic user interface, which receives operator's inputs and displays the apparatus outputs. For instance, the graphic user interface may include a touch screen, a display screen and hard keys for entering user's inputs or a combination thereof. During extracorporeal blood treatment, the control unit 100 is configured to control at least the pumps 11, 14, 17, 20, 20' to make sure that a prefixed patient net fluid removal is achieved in the course of a treatment time, as required by a prescription provided to the control unit 100, e.g. via the user interface.

The apparatus 1 comprises a main portion 22 comprising at least a casing for the control unit 100, the control unit 100, the user interface, the blood pump 11, the effluent pump 14, the dialysis fluid pump 17, the infusion pumps 20, 20' and devices to mount a disposable set (comprising the blood treatment unit 2, the extracorporeal blood circuit, the infusion circuit and the fluid circuit) to the main portion 22.

The apparatus for the extracorporeal treatment of blood 1 shown in Figure 1 further comprises a fluid diverting device 21 which is configured to be placed in-line between the main portion 22 of the apparatus 1 and the vascular access of a patient "P", in particular the access device 8 (e.g. a needle or a catheter) placed in fluid communication with the patient "P" vascular system. The fluid diverting device 21 may be placed in-line on the withdrawal line 6 and on the return line 7 and closer to the access device 8 than to the main portion 22 of the apparatus 1 for extracorporeal treatment of blood. The fluid diverting device 21 may be disposable, at least in part. The fluid diverting device 21 of Figure 1 is an add-on module which may be supplied to the apparatus and connected to the withdrawal line 6 and return line 7 at a later stage with respect to the manufacturing of the apparatus 1.

The fluid diverting device 21 comprises a substantially H-shaped conduits assembly comprising a withdrawal conduit 23, a return conduit 24 and a bridging conduit 25 connecting the withdrawal conduit 23 to the return conduit 24. Each of the withdrawal conduit 23, the return conduit 24 and the bridging conduit 25 of the embodiments shown in the annexed Figures is a straight plastic tube. The withdrawal conduit 23 is parallel to the return conduit 24 while the bridging conduit 25 is perpendicular to the withdrawal conduit 23 and to the return conduit 24.

The withdrawal conduit 23 has a first extremity 23a and an opposite second extremity 23b each provided with a quick connector, such as a Luer connector, to allow easy and quick connection/disconnection to/from the remaining portions of the withdrawal line 6. The first extremity 23a is connected or configured to be connected to a portion of the withdrawal line 6 comprising the pump section coupled to the blood pump 11. The second extremity 23b is connected or configured to be connected to a portion of the withdrawal line connected to the access device 8.

The return conduit 24 has a first extremity 24a and an opposite second extremity 24b each provided with a quick connector, such as a Luer connector, to allow easy and quick connection/disconnection to/from the remaining portions of the return line 7. The first extremity 24a is connected or configured to be connected to a portion of the return line 7 comprising the deaeration chamber 9 and the monitor valve 10. The second extremity 24b is connected or configured to be connected to a portion of the return line 7 connected to the access device 8.

The bridging conduit 25 has a first extremity 25a forming a junction to the withdrawal conduit 23 and an opposite second extremity 25b forming a junction to the return conduit 24. For instance, the bridging conduit 25, the withdrawal conduit 23 and the return conduit 24 are connected through T-connectors. The withdrawal conduit 23 comprises two branches connected to the T-connector. A branch comprising the first extremity 23a and a branch comprising the second extremity 23b. The return conduit 24 comprises two branches connected to the T-connector. A branch comprising the first extremity 24a and a branch comprising the second extremity 24b.

A plurality of valves (e.g. clamps) operate on the withdrawal conduit 23, on the return conduit 24 and on the bridging conduit 25 and are configured to divert a flow of liquid and/or blood without disconnecting the patient "P". The fluid diverting device 21 of the embodiments of Figures 1, 2 and 4 to 11 comprises a first withdrawal valve 26 and a second withdrawal valve 27 operating on the withdrawal conduit 23. The first withdrawal valve 26 is placed upstream, with respect to a flow of blood during treatment (flowing from the second extremity 23b to the first extremity 23a of the withdrawal conduit 23), the junction of the bridging conduit 25 to said withdrawal conduit 23 and the second withdrawal valve 27 is placed downstream, with respect to said flow of blood during treatment, the junction of the bridging conduit 25 to said withdrawal conduit 23. As disclosed in Figure 2, the first withdrawal valve 26 and the second withdrawal valve 27 are placed on opposite sides with respect to the T-connector joining the bridging conduit 25 to the withdrawal conduit 23.

The fluid diverting device 21 of the embodiments of Figures 1, 2 and 4 to 11 comprises a single return valve 28 operating on the return conduit 24. The return valve 28 is placed downstream the junction of the bridging conduit 25 to the return conduit 24 with respect to a flow of blood during treatment (flowing from the first extremity 24a to the second extremity 24b of the return conduit 24). As disclosed in Figure 2, the return valve 28 is placed at a side of the T-connector joining the bridging conduit 25 to the return conduit 24 and on the branch comprising the second extremity 24b of said return conduit 24.

In a variant embodiment, not shown, a first return valve and a second return valve operate on the return conduit 24 and are placed on opposite sides with respect to the T-connector joining the bridging conduit 25 to the return conduit 24, similar to the first withdrawal valve 26 and the second withdrawal valve 27. The return valve placed upstream the junction of the bridging conduit 25 to the return conduit 24 with respect to the flow of blood during treatment may perform the function of the monitor valve 10.

The fluid diverting device 21 of the embodiments of Figures 1, 2 and 4 to 11 comprises a first bridging valve 29 and a second bridging valve 30 operating on the bridging conduit 25. The first bridging valve 29 is placed closer to the return conduit 24 (closer than the second bridging valve 30) and the second bridging valve 30 is placed closer to the withdrawal conduit 23 (closer than the first bridging valve 29).

The bridging conduit 25 comprises a first branch having the first extremity 25a and a second branch having the second extremity 25b. The first branch and the second branch are connected to each other through a T-connector delimiting also an access point 31 suitable to connect a liquid source. The first bridging valve 29 is placed on the second branch bridging conduit 25 and the second bridging valve 30 is placed on the first branch of the bridging conduit 25. The access point 31 is located between the first bridging valve 29 and the second bridging valve 30.

A stretch of tubing departs form the access point 31 and is provided with an access valve 32. The stretch of tubing may be connected to a liquid source 33, like a syringe, e.g. of 50 ml, or a bag, e.g. of 500 ml. The liquid of said liquid source may be saline.

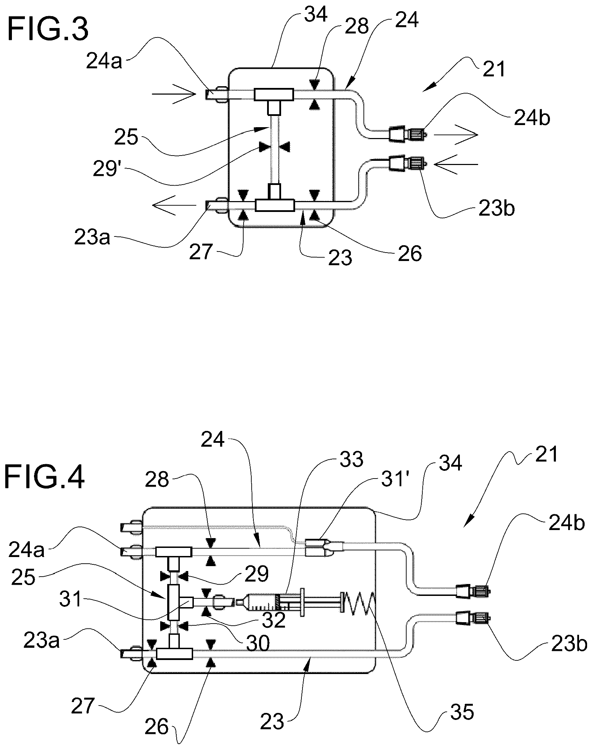

In the variant embodiment of Figure 3, the fluid diverting device 21 comprises a single bridging valve 29' operating on the bridging conduit 25 and does not comprise any access point 31. In the variant embodiment of Figure 4, the fluid diverting device 21 further comprises (in addition with respect to the embodiment of Figure 3) a further access point 31' on the return conduit 24, e.g. for calcium infusion. The further access point 31' is placed on the branch comprising the second extremity 24b and may be connected to a bag of the apparatus.

The substantially H-shaped conduits assembly and the plurality of valves 26, 27, 28, 29, 30, 32 are mounted on a holder 34 which may be shaped like a plate provided with seats, or other holding devices, configured to accommodate the substantially H-shaped conduits assembly and the plurality of valves 26, 27, 28, 29, 30, 32. In the embodiments of Figures 1, 2 and 4 to 11, the holder 34 comprises a seat, or other holding device to hold the syringe 33, and a spring 35 for pressurizing the syringe 33 and ejecting the fluid contained therein through the access point 31.

The fluid diverting device 21 may also comprise one or more sensors (e.g. pressure sensor, flowmeter, air detector, etc.), not shown, active on the H-shaped conduits assembly, i.e. on the return conduit 24 and/or on the withdrawal conduit 23, on the bridging conduit 25.

The plurality of valves 26, 27, 28, 29, 30, 32 and the sensor/s are operatively connected or connectable to the control unit 100 of the apparatus 1 and/or to a control unit of the fluid diverting device 21 which may be slaved to the control unit 100 of the apparatus 1. Said control unit/s is/are configured for commanding, optionally automatically, the configurations of the plurality of valves 26, 27, 28, 29, 30, 32. The valves 26, 27, 28, 29, 30, 32 and/or the control unit of the fluid diverting device 21 may be connected to the control unit 100 of the apparatus 1 through a wired or wireless communication device.

The fluid diverting device 21 comprises a power supply to power the valves 26, 27, 28, 29, 30, 32 and/or the sensor/s and/or control unit or said valves 26, 27, 28, 29, 30, 32 and/or the control unit of the fluid diverting device 21 may be connected to a power supply of the apparatus 1 to be powered by said apparatus 1.

The fluid diverting device 21 may be a portable module and/or comprises an attachment device configured to install said fluid diverting device on another element, optionally in a removable manner, e.g. to clothing of a patient "P" or to a bed for the patient "P" or to a main portion of the apparatus for extracorporeal treatment of blood. The lengths of the portion of the return line 7 comprising the deaeration chamber 9 and the monitor valve 10 and of the portion of the withdrawal line 6 comprising the pump section coupled to the blood pump 11 are such to place the fluid diverting device close to the patient "P".

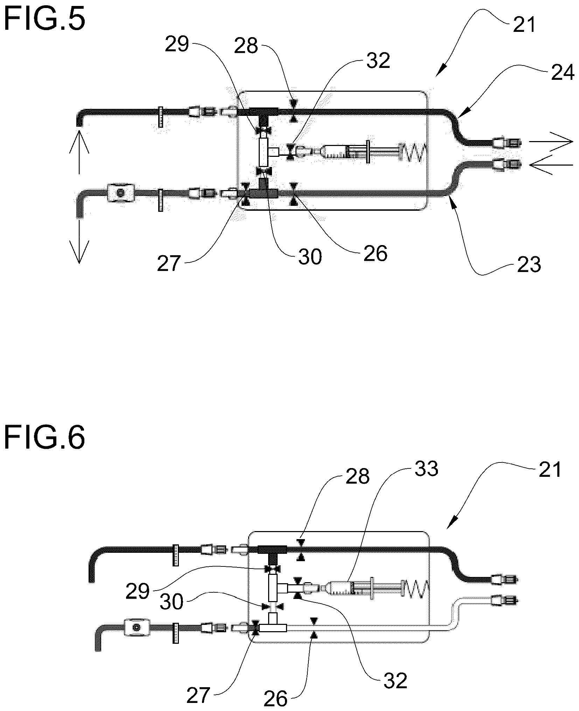

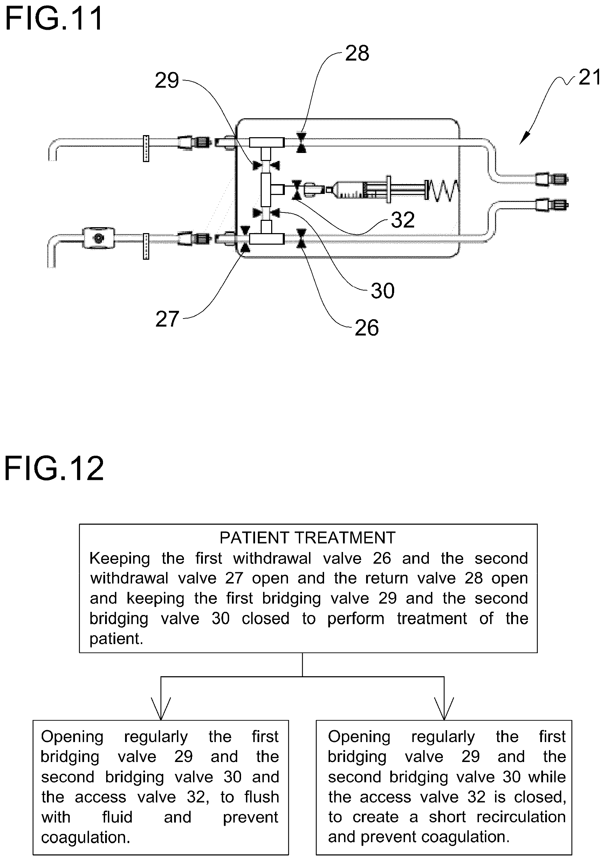

In use and according to a method of the invention for diverting a flow of liquid and/or blood in the apparatus 1, before treatment of the patient "P", the blood circuit is primed and the control unit 100 controls the valves 26, 27, 28, 29, 30, 32 to allow priming the withdrawal conduit 23, the return conduit 24 and the bridging conduit 25, using either solution bags of the apparatus 1 or saline from the liquid source 33 connected to the access point 31. During patient treatment (Figures 5 and 12), when blood flows from patient "P" through the withdrawal line 6, into the primary chamber 3 of the blood treatment unit 2 and then back into the patient "P" through the return line 7, the control unit 100 controls the valves 26, 27, 28, 29, 30, 32 to configure said valves 26, 27, 28, 29, 30, 32 in a treatment configuration. In the treatment configuration, the first bridging valve 29, the second bridging valve 30 and the access valve 32 are closed, the first withdrawal valve 26, the second withdrawal valve 27 and the return valve 28 are open.

During patient treatment, since blood may stagnate at the closed first bridging valve 29 and the second bridging valve 30, in order to prevent coagulation, the first bridging valve 29 and the second bridging valve 30 and the access valve 32 are opened regularly (intermittently), to flush the bridging conduit 25 with fluid and prevent coagulation. According to a variant of the method, the first bridging valve 29 and the second bridging valve 30 are opened regularly (intermittently) while the access valve 32 is closed, to flush the bridging conduit 25 with blood, creating a short recirculation and preventing coagulation (Figure 12).

In case of Access Extremely Negative (AEN) alarm, the blood pump 11 is stopped, the second bridging valve 30 and the access valve 32 are opened to enable saline to be sucked from the syringe 33 until access pressure reaches an approximately atmospheric level or the first bridging valve 29 and the second bridging valve 30 are both opened to create connection between the withdrawal conduit 23 and the return conduit 24 and to normalize pressure.

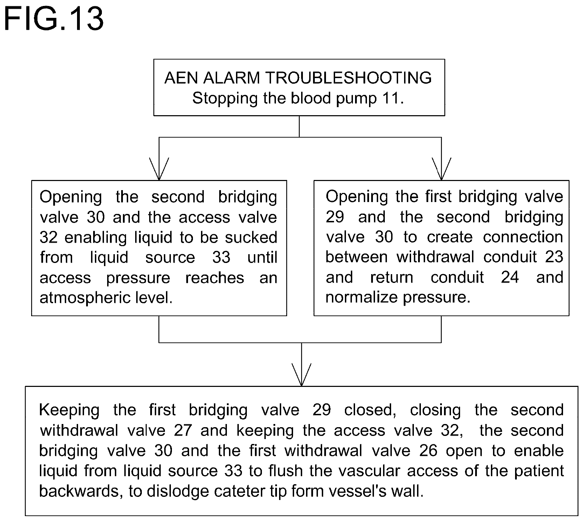

After the access pressure got closer to the atmospheric level, the first bridging valve 29 is kept closed, the second withdrawal valve 27 is closed while the access valve 32, the second bridging valve 30 and the first withdrawal valve 26 are kept open. This way, the fluid from syringe 33 flushes the vascular access of the patient "P" backwards to help dislodge the catheter tip from vessel's wall (Figures 6 and 13).

According to another possible action to solve an AEN alarm, the first withdrawal valve 26 and the second withdrawal valve 27 are closed while the other valves are open. The blood pump 11 is controlled to turn in order to increase the access negative pressure till a defined value and then stopped. Then the first withdrawal valve 26 and the second withdrawal valve 27 are opened to create a sudden negative pressure peak in the catheter's access way to move or break a clot.

If these attempts fail, the control unit 100 controls the plurality of valves to 26, 27, 28, 29, 30, 32 to engage blood recirculation, while notifying the staff that action is required (moving the patient "P", caring for the vascular access, etc.).

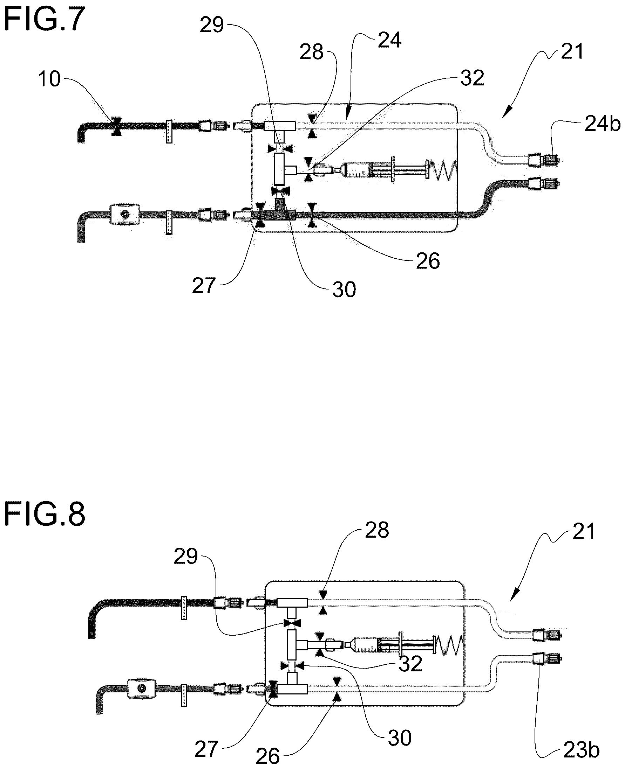

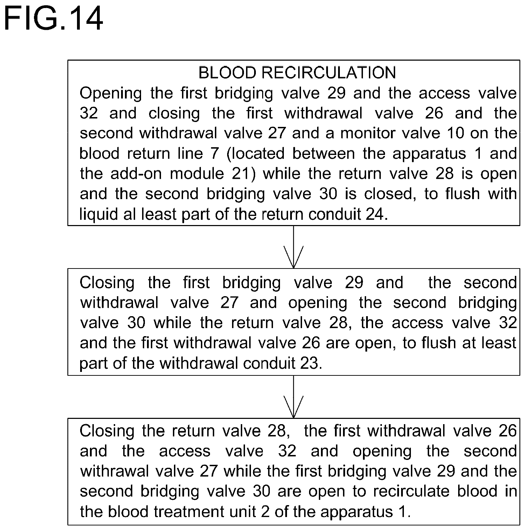

Prior to enabling blood recirculation, flushing is needed in the return conduit 24 and withdrawal conduit 23 to avoid clotting and to enable patient reconnection after staff intervention. To this aim, the branch of the return conduit 24 comprising the second extremity 24b is flushed with fluid by closing the second bridging valve 30 and the monitor valve 10 while the first bridging valve 29, the first withdrawal valve 26, the second withdrawal valve 27, the return valve 28 and the access valve 32 are open and fluid from the syringe 33 is injected (Figures 7 and 14).

The branch of the withdrawal conduit 6 having the second extremity 23b is then flushed. The first bridging valve 29 and the second withdrawal valve 27 are closed, the second bridging valve 30, the first withdrawal valve 26 and the access valve 32 are open and saline from the syringe 33 is injected (Figures 8 and 14).

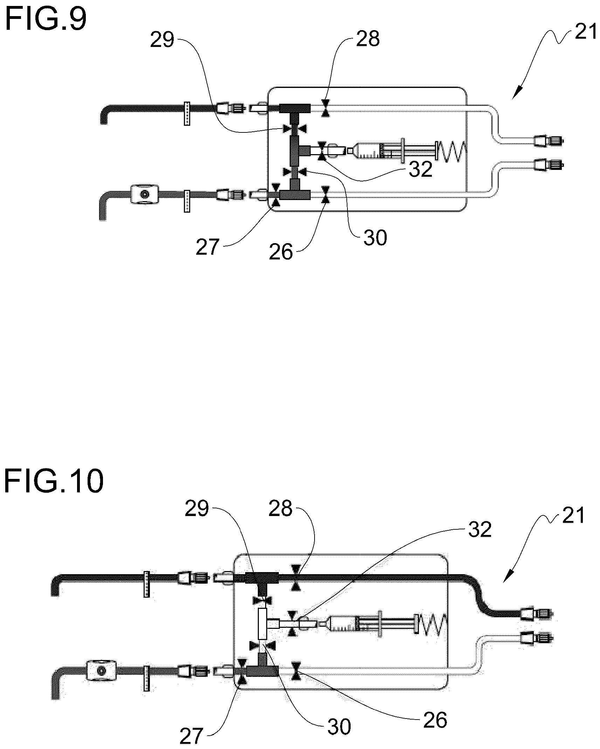

The blood recirculation starts with the return valve 28 closed, the first withdrawal valve 26 closed, the second withdrawal valve 27 open and the first and second bridging valves 29, 30 open, so that blood recirculates in the blood treatment unit 2 of the apparatus 1 and staff intervention is executed (Figures 9 and 14).

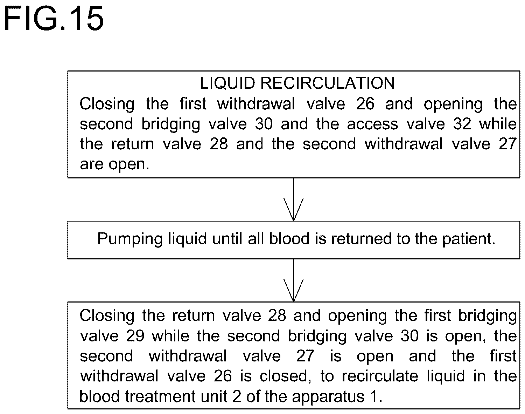

At the end of the treatment, with blood return to the patient "P", saline recirculation starts. With the first withdrawal valve 26 and the first bridging valve 29 closed and the second bridging valve 30, the return valve 28, the second withdrawal valve 27 and the access valve 32 open, saline is pumped by the blood pump 11 until all blood is returned to the patient "P". Then, the return valve 28 is closed and the first bridging valve 29 is opened while the second bridging valve 30 is still open, the second withdrawal valve 27 is open and the first withdrawal valve 26 is closed, to recirculate liquid in the blood treatment unit 2 of the apparatus 1 (Figures 11 and 15).

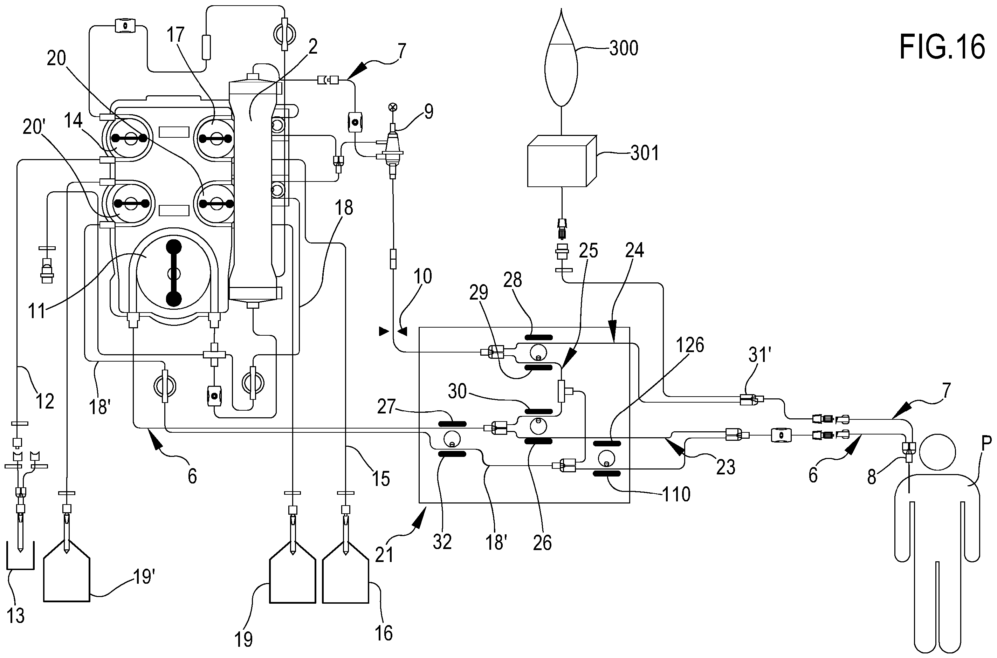

Figures 16 to 20B show another embodiment of the fluid diverting device 21. The fluid diverting device 21 of this embodiment may be mounted on a side of the main portion 22 of the apparatus 1.

The fluid diverting device 21 comprises the first withdrawal valve 26, the second withdrawal valve 27, the return valve 28, the first bridging valve 29, the second bridging valve 30 and the access point 31 as the embodiment of Figures 1, 2 and 4 - 11.

The type of valves is different from the embodiment of Figures 1, 2 and 4 -11. Each of the plurality of valves is a rotating clamp or pinch.

The first bridging valve 29 and the return valve 28 share a common rotating body 140 and comprise two respective abutment elements 145, one for the first bridging valve 29 and one for the return valve 28. The second bridging valve 30 and the first withdrawal valve 26 share a common rotating body 140 and two respective abutment elements 145, one for the second bridging valve 30 and one for first withdrawal valve 26.

Figure 16 also shows a calcium bag 300 connected to the further access point 31' on the return conduit 24 for calcium infusion operated through a calcium pump 301.

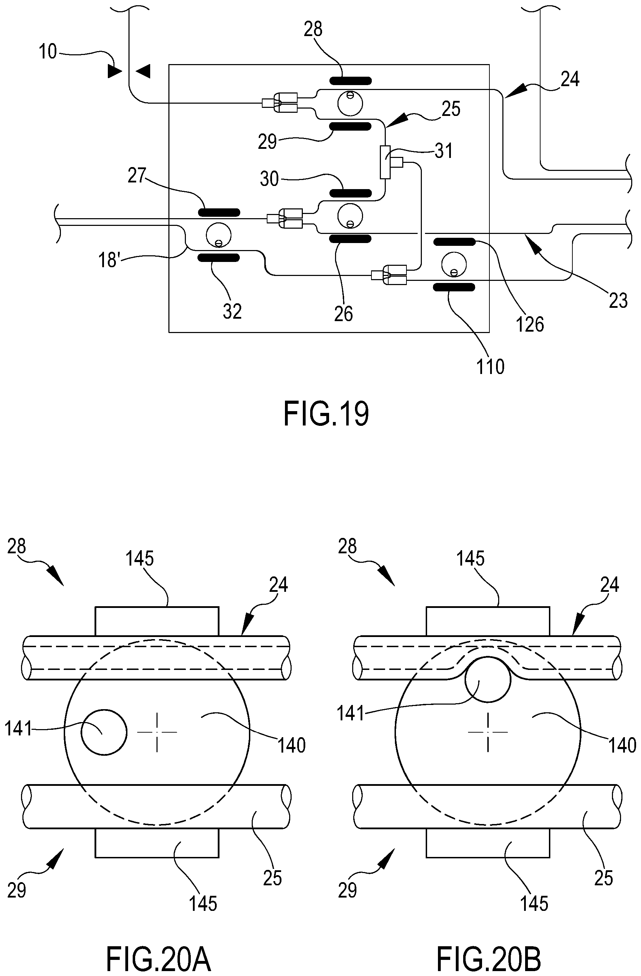

Figures 20A and 20B show an enlarged view of the first bridging valve 29 and the return valve 28. Each valve comprises the rotating body 140 and one abutment element 145. The rotating body 140 comprises a presser 141 spaced form a rotation axis of the rotating body 140. The presser 141 is placed close to the rotating body 140 such that a tube section may be positioned between the abutment element 145 and the presser 141. The assembly comprising the first bridging valve 29 and the return valve 28 shown in Figures 20A and 20B comprises one rotating body 140 and two abutment elements 145. Said two abutment elements 145 are stationary with respect to the rotation axis of the rotating body 140 and are positioned on opposite sides with respect to the rotating body 140. A tube section of the return conduit 24 is placed against one of the abutment elements 145 and a tube section of the bridging conduit 25 is placed against the other of the abutment elements 145.

The rotating body 140 is rotatable between a first clamping position (one of the two valves is closed and the other is open), a second clamping position (one of the two valves is closed and the other open) and a release position (both valves are open).

In the first clamping position (Figure 20B), the presser 141 of the rotating element 140 is close to one of the two abutment elements 145 to press and close a portion of a tube (the return conduit 24 in Figure 20B) between said rotating element 140 and said abutment element 145.

In the second clamping position (not show), the presser 141 of the rotating element 140 is close to the other of the two abutment elements 145 to press and close a portion of another tube (it should be the bridging conduit 25 in Figures 20A and 20B) between said rotating element 140 and said abutment element 145.

In the release position, the presser 141 of the rotating element is spaced from both the abutment elements 145, to open both portions of tubes (Figure 20A).

The assembly comprising the first withdrawal valve 26 and the second bridging valve 30 has the same structure and functionality shown in Figures 20A and 20B and disclosed above.

The access point 31 of this embodiment is connected to the pre-blood pump infusion line 18' with its own pre-blood pump infusion fluid container 19' and infusion pump 20'. The pre-blood pump infusion line 18' has a branch connected to the access point 31 and a branch connected to the blood withdrawal line 6 at a site between the vascular access 8 and the fluid diverting device 21.

The infusion pump 20' operates on the pre-blood pump infusion line 18' to regulate respective flow rates through the cited branches.

A first infusion valve or access valve 32 is placed upstream the branch of the pre-blood pump infusion line 18' connected to the access point 31. A second infusion valve 110 is placed downstream the branch connected to the access point 31, i.e. on the branch connected to the blood withdrawal line 6.

Also the access valve 32 is a rotating clamp or pinch. Like the assembly comprising the first withdrawal valve 26 and the second bridging valve 30 and the assembly comprising the first bridging valve 29 and the return valve 28, also the access valve 32 is coupled to the second withdrawal valve 27. The second withdrawal valve 27 and the access valve 32 comprise one common rotating body 140 and two respective abutment elements 145.

An auxiliary withdrawal valve 126 operates on the withdrawal conduit 6 and is placed downstream, with respect to a flow of blood during treatment, the first withdrawal valve 26. The second infusion valve 110 and the auxiliary withdrawal valve 126 comprise one common rotating body 140 and two respective abutment elements 145.

As shown in Figures 16 to 20B, the common rotating body 140 of the first bridging valve 29 and return valve 28 is placed between the return conduit 24 and the bridging conduit 25, the common rotating body 140 of the first withdrawal valve 26 and second bridging valve 30 is placed between the withdrawal conduit 23 and the bridging conduit 25, the common rotating body 140 of the access valve 32 and withdrawal valve 27 is placed between the withdrawal conduit 23 and the pre-blood pump infusion line 18', the common rotating body 140 of the second infusion valve 110 and auxiliary withdrawal valve 126 is placed between the withdrawal conduit 23 and the pre-blood pump infusion line 18'.

The position of the valves 26, 27, 28, 29, 30, 32, 110, 126 configured to perform patient treatment is shown in Figure 16. The first bridging valve 29, the second bridging valve 30, the access valve 32 and the second infusion valve 110 are closed, the first withdrawal valve 26, the second withdrawal valve 27, the auxiliary withdrawal valve 126 and the return valve 28 are open.

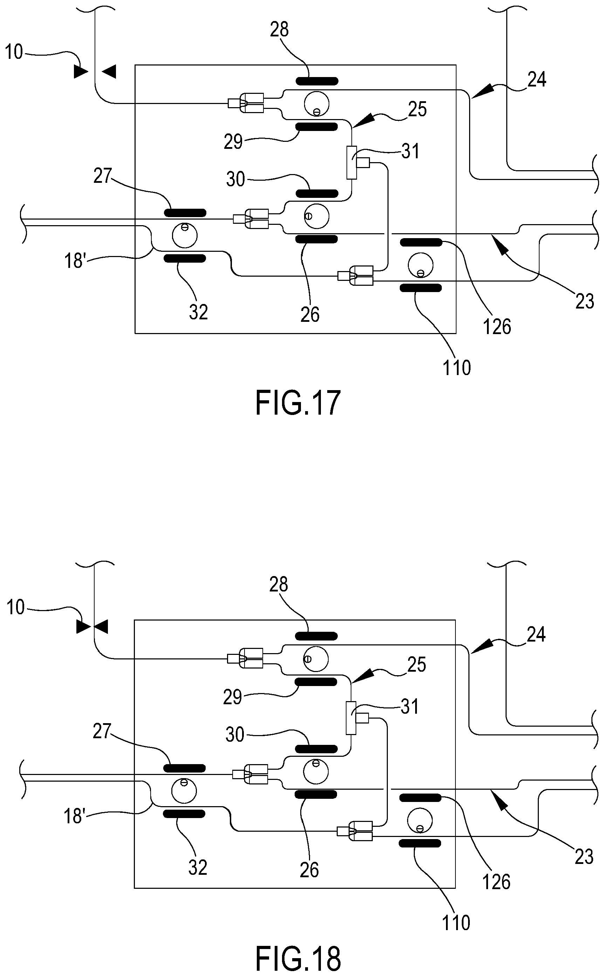

The position of the valves 26, 27, 28, 29, 30, 32, 110, 126 configured to perform flushing of the withdrawal conduit 23 and of the vascular access of the patient "P" is shown in Figure 17. The first bridging valve 29, the second withdrawal valve 27 and the second infusion valve 110 are closed while the access valve 32, the second bridging valve 30, the auxiliary withdrawal valve 126 and the first withdrawal valve 26 are open. Fluid from the pre-blood pump infusion fluid container 19' flows through the access point 31 into the withdrawal conduit 23 and towards to patient "P".

The position of the valves 26, 27, 28, 29, 30, 32, 110, 126 configured to perform flushing of the return conduit 24 is shown in Figure 18. The first bridging valve 29, the return valve 28, the access valve 32, the first withdrawal valve 26 and the auxiliary withdrawal valve 126 are open while the second withdrawal valve 27, the second bridging valve 30 and the second infusion valve 110 are closed. Fluid from the pre-blood pump infusion fluid container 19' flows through the access point 31 into the return conduit 24 and towards to patient "P".

The position of the valves 26, 27, 28, 29, 30, 32, 110, 126 configured to perform blood recirculation in the blood treatment unit 2 of the apparatus 1 is shown in Figure 19.

The return valve 28, the first withdrawal valve 26, the second infusion valve 110 and the access valve 32 are closed while the second withdrawal valve 27, the first and second bridging valves 29, 30 and the auxiliary withdrawal valve 126 are open.

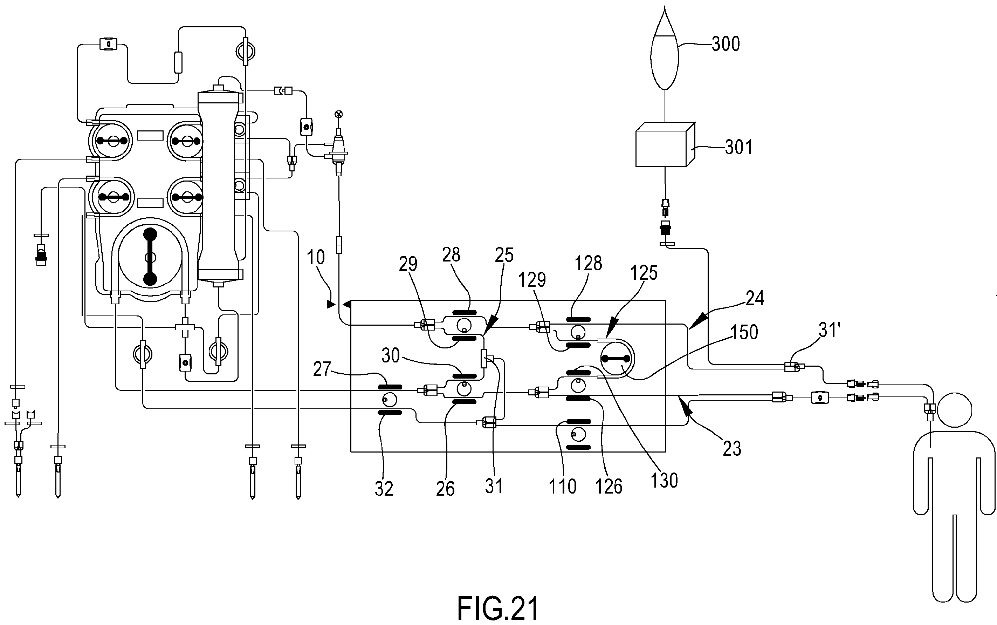

Figures 21 to 26 show a variant of the embodiment Figures 16 to 20B. With respect to Figures 16 to 20B, this variant additionally comprises an auxiliary bridging conduit 125 which is arranged in parallel with respect to the bridging conduit 25 and also connects or is configured to connect the withdrawal conduit 23 to the return conduit 24. The auxiliary bridging conduit 125 is positioned between the bridging conduit 25 and the access device 8. With respect to a flow of blood during treatment, the auxiliary bridging conduit 125 is connected to the withdrawal conduit 23 at a connection site placed upstream the bridging conduit 25 and is connected to the return conduit 6 at a connection site placed downstream the bridging conduit 25.

An auxiliary pump 150, e.g. a peristaltic pump, operates on the auxiliary bridging conduit 125 and is configured to recirculate blood on a patient "P" side.

The auxiliary withdrawal valve 126 operates on the withdrawal conduit 23 like in Figures 16 to 20B and an auxiliary return valve 128 operates on the return conduit 24. The auxiliary return valve 128 is placed downstream, with respect to a flow of blood during treatment, the return valve 28. With respect to said flow of blood during treatment, the auxiliary withdrawal valve 126 is placed upstream the connection site of the auxiliary bridging conduit 125 to the withdrawal conduit 23 and the auxiliary return valve 128 is placed downstream the connection site of the auxiliary bridging conduit 125 to the return conduit 24.

A first auxiliary bridging valve 129 and a second auxiliary bridging valve 130 operate on the auxiliary bridging conduit 125 and are placed on opposite sides with respect to the auxiliary pump 150.

The first auxiliary bridging valve 129 and the auxiliary return valve 128 comprise one common rotating body 140 and two respective abutment elements 145. Likewise, the auxiliary withdrawal valve 126 and the second auxiliary bridging valve 130 comprise one common rotating body 140 and two respective abutment elements 145.

As shown in Figures 21 to 25, the common rotating body 140 of the first auxiliary bridging valve 129 and auxiliary return valve 128 is placed between the return conduit 24 and the auxiliary bridging conduit 125. The common rotating body 140 of the auxiliary withdrawal valve 126 and the second auxiliary bridging valve 130 is placed between the withdrawal conduit 23 and the auxiliary bridging conduit 125.

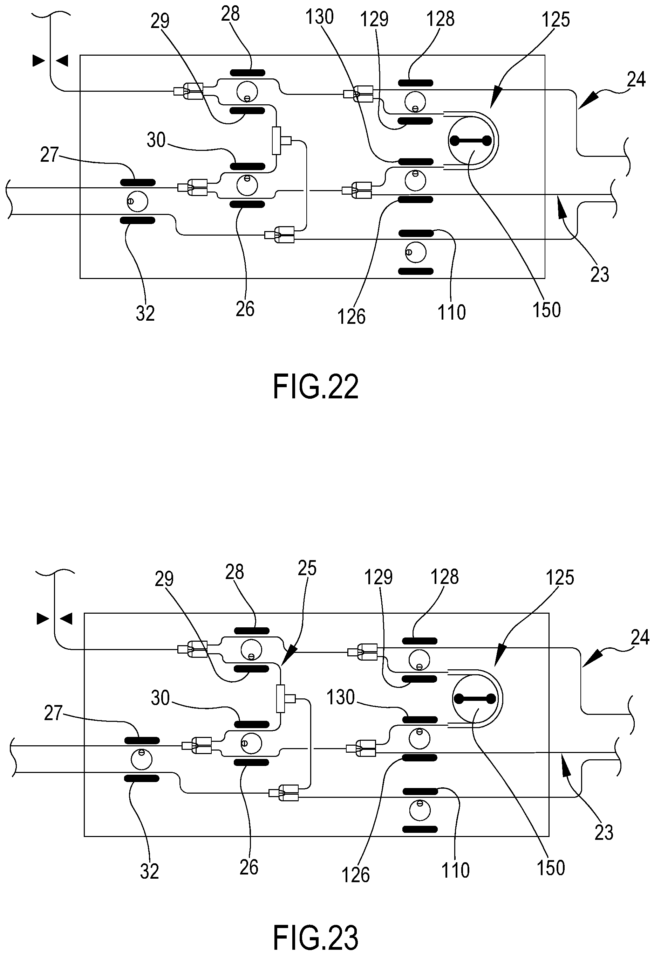

During patient treatment (Figures 21 and 22), the first auxiliary bridging valve 129 and the second auxiliary bridging valve 130 are closed while the auxiliary withdrawal valve 126 and the auxiliary return valve 128 are open. The position of the other valves may the same as shown in Figure 16. The access valve 32 may be open and the pre-blood pump infusion line 18' works to supply replacement fluid from the pre-blood pump infusion fluid container 19' to the blood withdrawal line 6.

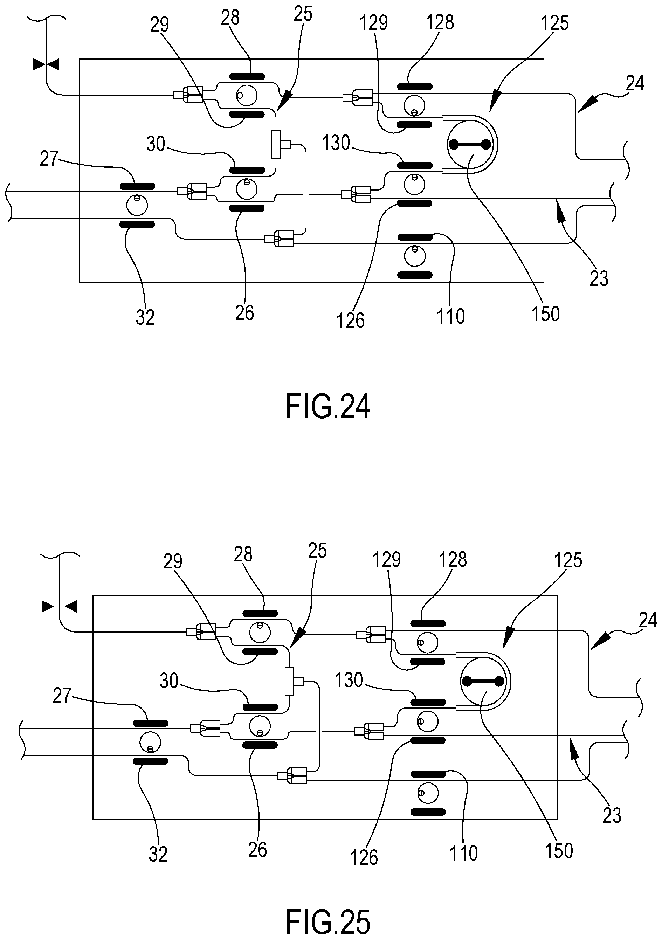

When flushing the withdrawal conduit 23 (Figure 23) or the return conduit 24 (Figure 24), the auxiliary withdrawal valve 126 and the auxiliary return valve 128 are open while the first auxiliary bridging valve 129 and the second auxiliary bridging valve 130 are closed. The position of the other valves may the same as shown respectively in Figure 17 or Figure 18.

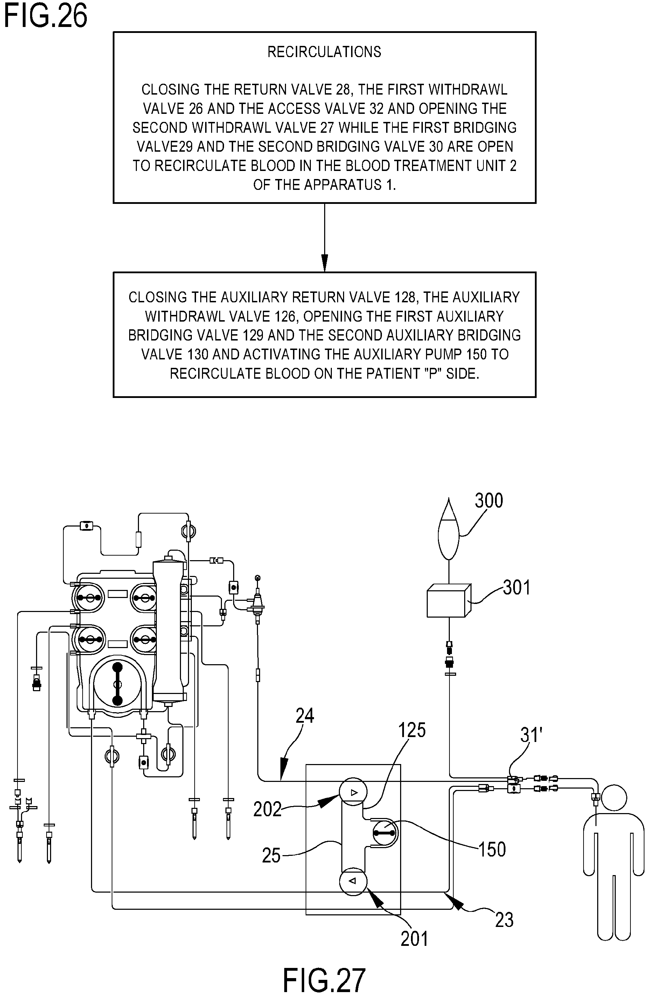

During blood recirculation (Figures 25 and 26), the auxiliary withdrawal valve 126, the auxiliary return valve 128, the first auxiliary bridging valve 126 and the second auxiliary bridging valve all are open. The position of the other valves may the same as shown in Figure 19. The blood pump 11 works to recirculate blood through the treatment unit 2 and the auxiliary pump 150 is activated to recirculate blood on the patient "P" side.

Figures 27 to 30 show another different embodiment of the fluid diverting device 21. The fluid diverting device 21 of this embodiment comprises distributors 201, 202 in place of valves. The distributors 201, 202 may be disposable. The distributors 201, 202 may be rotary distributors and may be operated through rotary actuators, not shown, part of the main portion 22 and controlled by the control unit 100.

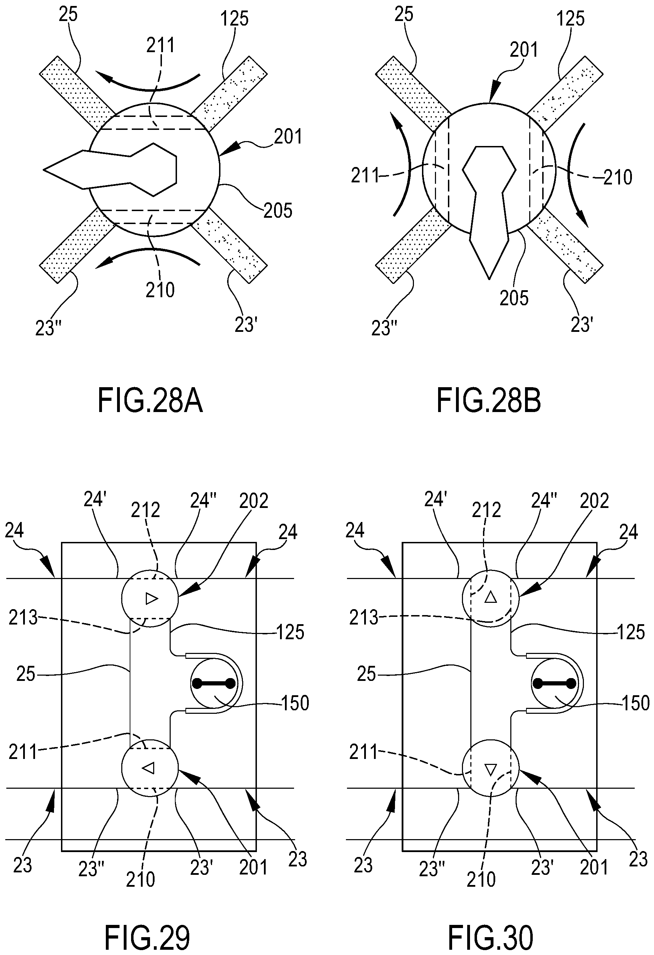

The fluid diverting device 21 comprises (Figures 28 and 29) the withdrawal conduit 23, the return conduit 24, the bridging conduit 25 and the auxiliary bridging conduit 125. The withdrawal conduit 23 comprises a first branch 23' and a second branch 23". Likewise, the return conduit 24 comprises a first branch 24' and a second branch 24". The auxiliary bridging conduit 125 is coupled to the auxiliary pump 150.

A first distributor 201 is operationally coupled to the withdrawal conduit 23, to the bridging conduit 25 and to the auxiliary bridging conduit 125. The first distributor 201 is operative between the first branch 23' and the second branch 23" of the withdrawal conduit 23. The first distributor 201 is also operative between a first end of the bridging conduit 25 and a first end of the auxiliary bridging conduit 125.

A second distributor 202 is operationally coupled to the return conduit 24, to the bridging conduit 25 and to the auxiliary bridging conduit 125. The second distributor 202 is operative between the first branch 24' and the second branch 24" of the return conduit 24. The second distributor 202 is also operative between a second end of the bridging conduit 25 and a second end of the auxiliary bridging conduit 125.

As better shown in Figures 28A and 28B, the first distributor 201 comprises a rotating element 205 provided with a first through passage 210 and a second through passage 211. The first through passage 210 and the second through passage 211 are parallel and each has open ends facing on opposite parts of the rotating element 205. The second distributor 202 comprises a rotating element 205 provided with a first through passage 212 and a second through passage 213. The structure of the second distributor 202 (not shown in an enlarged view) is the same as the first distributor 201.

Each of the first distributor 201 and the second distributor 202 may be rotated between: a first configuration corresponding to the treatment configuration and a second configuration corresponding to the recirculation configuration.

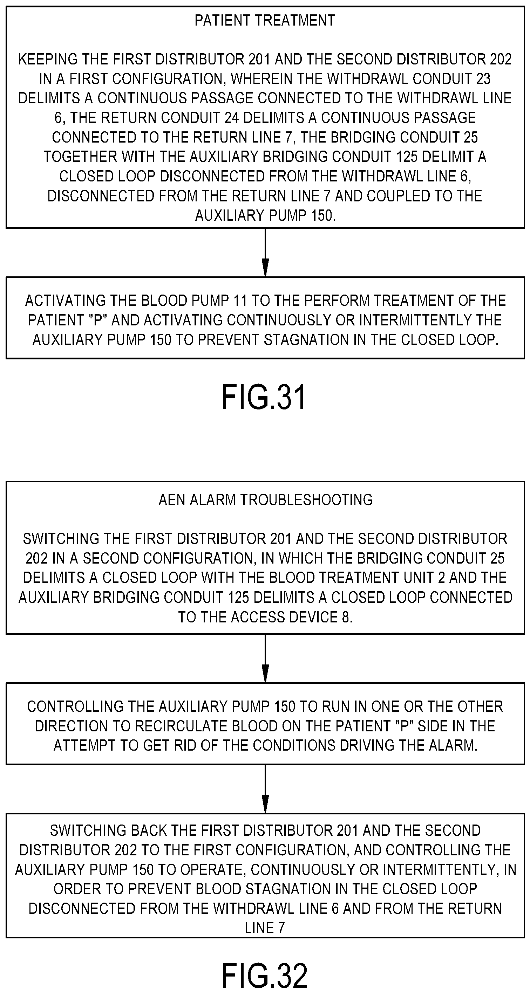

In the first configuration of Figures 27, 28A, 29 and 31, the withdrawal conduit 23 delimits a continuous passage connected to the withdrawal line 6.

The first through passage 210 of the first distributor 201 connects the first branch 23' and the second branch 23" of the withdrawal conduit 23 and the second through passage 211 of the first distributor 201 connects the first end of the bridging conduit 25 to the first end of the auxiliary bridging conduit 125.

In this first configuration, the return conduit 24 delimits a continuous passage connected to the return line 7 (Figure 29).

The first through passage 212 of the second distributor 202 connects a first branch 24' and a second branch 24" of the return conduit 24 and the second through passage 213 of the second distributor 202 connects the second end of the bridging conduit 25 to the second end of the auxiliary bridging conduit 125.

In this first configuration, the bridging conduit 25 together with the auxiliary bridging conduit 125 delimit a closed loop disconnected from the withdrawal line 6, disconnected from the return line 7 and coupled to the auxiliary pump 150.

In the second configuration of Figures 28B, 30 and 32, the bridging conduit 25 delimits a closed loop with the blood treatment unit 2 to recirculate blood in the blood treatment unit 2 and the auxiliary bridging conduit 125 delimits a closed loop connected to the access device 8, to recirculate blood on the patient "P" side. The closed loop on the side of the blood treatment unit 2 is disconnected from the closed loop on the patient "P" side.

In this second configuration, the first through passage 210 of the first distributor 201 connects the first branch 23' of the withdrawal conduit 23 to the auxiliary bridging conduit 125 and the second through passage 211 of the first distributor 201 connects the second branch 23" of the withdrawal conduit 23 to the bridging conduit 25 (Figures 28B and 30).

In this second configuration, the first through passage 212 of the second distributor 202 connects the first branch 24' of the return conduit 24 to the bridging conduit 25 and the second through passage 213 of the second distributor 202 connects the second branch 24" of the return conduit 24 to the auxiliary bridging conduit 125 (Figure 30).

In case of Access Extremely Negative (AEN) alarm during blood treatment, the control unit 100 switches the first and second distributors 201, 202 from the first configuration to the second configuration. The auxiliary pump 150 is controlled to run in one or the other direction in the attempt to get rid of the conditions driving the alarm. Then the distributors 201, 202 would switch back to the first configuration (treatment mode), and the auxiliary pump 150 would operate at low rate, continuously or intermittently, in order to prevent blood stagnation.

While the invention has been described in connection with what is presently considered to be the most practical and preferred embodiments, it is to be understood that the invention is not to be limited to the disclosed embodiments, but on the contrary, is intended to cover various modifications and equivalent arrangements included within the scope of the appended claims.

## Claims

1. Fluid diverting device for an apparatus for extracorporeal treatment of blood, wherein the apparatus (1) comprises:
- a blood treatment unit (2);
- an extracorporeal blood circuit coupled to the blood treatment unit (2) and comprising a blood withdrawal line (6) and a blood return line (7) connectable to a vascular access of a patient (P);
- a blood pump (11) configured to be coupled to a pump section of the extracorporeal blood circuit;
wherein the fluid diverting device (21) is configured to be placed in-line between a blood set of the apparatus (1) and the vascular access of the patient (P) and comprises:
- a substantially H-shaped conduits assembly comprising a withdrawal conduit (23), a return conduit (24) and at least one bridging conduit (25, 125) connecting or configured to connect the withdrawal conduit (23) to the return conduit (24); wherein the withdrawal conduit (23) is connected or connectable upstream and downstream to the withdrawal line (6), wherein the return conduit (24) is connected or connectable upstream and downstream to the return line (7);
- a plurality of distributors (201, 202) operating on the withdrawal conduit (23), on the return conduit (24) and on the at least one bridging conduit (25) and configured to divert a flow of liquid and/or blood without disconnecting the patient (P);
- an auxiliary bridging conduit (125) connecting or configured to connect the withdrawal conduit (23) to the return conduit (24) and arranged in parallel with respect to the bridging conduit (25);
wherein the plurality of distributors (201, 202) comprises:
- a first distributor (201) operatively coupled to the withdrawal conduit (23), to the bridging conduit (25) and to the auxiliary bridging conduit (125);
- a second distributor (202) operatively coupled to the return conduit (24), to the bridging conduit (25) and to the auxiliary bridging conduit (125).

2. The device according to claim 1, wherein the first distributor (201) and the second distributor (202) are movable between:
a first configuration, in which the withdrawal conduit (23) delimits a continuous passage connected to the withdrawal line (6), the return conduit (24) delimits a continuous passage connected to the return line (7) and the bridging conduit (25) together with the auxiliary bridging conduit (125) delimit a closed loop disconnected from the withdrawal line (6) and the return line (7); and
a second configuration, in which the bridging conduit (25) delimits a closed loop with the blood treatment unit (2) to recirculate blood in the blood treatment unit (2) and in which the auxiliary bridging conduit (125) delimits a closed loop connected to the access device (8).

3. The device according to claim 1 or 2, wherein each of the first distributor (201) and the second distributor (202) comprises a rotating element (205) provided with through passages (210, 211, 212, 213) configured to communicate with branches of the withdrawal conduit (23) or of the return conduit (24) and with the bridging conduit (25) and the auxiliary bridging conduit (125).

4. The device according to claim 3 when according to claim 2, wherein the through passages comprise a first through passage (210) and a second through passage (211);
wherein, in the first configuration, the first through passage (210) of the first distributor (201) connects a first branch (23') and a second branch (23") of the withdrawal conduit (23) and the second through passage (211) of the first distributor (201) connects the bridging conduit (25) to the auxiliary bridging conduit (125);
wherein, in the first configuration, the first through passage (212) of the second distributor (202) connects a first branch (24') and a second branch (24") of the return conduit (24) and the second through passage (213) of the second distributor (202) connects the bridging conduit (25) to the auxiliary bridging conduit (125).

5. The device according to claim 4,
wherein, in the second configuration, the first through passage (210) of the first distributor (201) connects a first branch (23') of the withdrawal conduit (23) to the auxiliary bridging conduit (1025) and the second through passage (211) of the first distributor (201) connects a second branch (23") of the withdrawal conduit (23) to the bridging conduit (25);
wherein, in the second configuration, the first through passage (212) of the second distributor (202) connects a first branch (24') of the return conduit (24) to the bridging conduit (25) and the second through passage (213) of the second distributor (202) connects a second branch (24") of the return conduit (24) to the auxiliary bridging conduit (125).

6. The device according to any of claims 1 to 5, wherein an auxiliary pump (150) is operating on the auxiliary bridging conduit (125) and is at least configured to recirculate blood on a patient side.

7. The device according to claim 6, comprising an auxiliary withdrawal valve (126) operating on the withdrawal conduit (23) and an auxiliary return valve (128) operating on the return conduit (24), a first auxiliary bridging valve (129) and a second auxiliary bridging valve (130) operating on the auxiliary bridging conduit (125) and placed on opposite sides with respect to the auxiliary pump (150).

8. The device according to any of claims 1 to 7, wherein the withdrawal conduit (23) is substantially parallel to the return conduit (24) and wherein the bridging conduit (25) is at least in part transversal, optionally perpendicular, to the withdrawal conduit (23) and to the return conduit (24).

9. The device according to any of claims 1 to 8, wherein at least one of the withdrawal conduit (23), the return conduit (24) and the bridging conduit (25) is at least in part straight.

10. A blood set for an apparatus for extracorporeal treatment of blood, the blood set comprising:
- a blood treatment unit (2);
- an extracorporeal blood circuit coupled to the blood treatment unit (2) and comprising a blood withdrawal line (6) and a blood return line (7) connectable to a vascular access of a patient (P), optionally each of the blood withdrawal line (6) and the blood return line (7) having a respective end connector configured to be connected to the vascular access of the patient (P);
- a fluid diverting device (21) according to one of the preceding claims 1 to 9, wherein the fluid diverting device (21) is placed or is configured to be placed in-line between the blood set of the apparatus (1) for extracorporeal treatment of blood and the vascular access of the patient (P).

11. The blood set of claim 10, wherein the withdrawal conduit (23) and the return conduit (24) have a respective end counter-connector configured to be coupled or coupled with the end connector of the blood withdrawal line (6) and of the blood return line (7).
